(19) Europäisches Patentamt
European Patent Office
Office européen des brevets

(11) Veröffentlichungsnummer: **0 044 959**
**A1**

# (12) EUROPÄISCHE PATENTANMELDUNG

(21) Anmeldenummer: **81105048.3**

(22) Anmeldetag: **30.06.81**

(51) Int. Cl.³: **A 61 K 31/77**, A 61 K 31/785

(30) Priorität: **12.07.80 DE 3026448**

(43) Veröffentlichungstag der Anmeldung: **03.02.82**
**Patentblatt 82/5**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Bömer, Bruno, Dr., Gustav-Freytag-Strasse 2, D-5090 Leverkusen 1 (DE)**
Erfinder: **Wolf, Gerhard Dieter, Dr., Wilhelm-Busch-Strasse 29, D-4047 Dormagen 5 (DE)**
Erfinder: **Bierling, Robert, Dr., Merlinweg 18 b, D-5600 Wuppertal 1 (DE)**

## (54) Antitumoral wirkende Mittel.

(57) Polyätherpolyole und Derivate von Polyätherpolyolen der allgemeinen Formel

$$R-\left[(O-CH-CH_2)_n-OR'\right]_m$$

in welcher

A = statistisch verteilt H oder $CH_3$, wobei maximal 50% der A-Gruppen $CH_3$ sind,

n = Zahlen von 2 bis 500,

m = Zahlen von 2 bis 8,

R = Rest eines Diols oder Polyols mit bis zu 8 OH-Gruppen und 2–12 C-Atomen,

die Reste R' gleich oder verschieden sind, und Wasserstoff, einen aliphatischen, alicyclischen, aromatischen oder heterocyclischen Rest mit 1–8 C-Atomen, einen Rest

X–CO– mit X = Wasserstoff, aliphatischer, alicyclischer oder aromatischer Rest mit 1–7 C-Atomen, einen Rest

Y–O–CO– mit Y = aliphatischer, cycloaliphatischer, aromatischer oder heterocyclischer Rest mit 1–7 C-Atomen, oder einen Rest

Z–NH–CO– mit Z = Wasserstoff, aliphatischer, cycloaliphatischer, aromatischer oder heterocyclischer Rest mit 1–7 C-Atomen

bedeuten, besitzen starke antitumorale Eigenschaften.

EP 0 044 959 A1

BAYER AKTIENGESELLSCHAFT 5090 Leverkusen-Bayerwerk
Zentralbereich
Patente, Marken und Lizenzen Si/AB/mo/kl-c

## Antitumoral wirkende Mittel

Die vorliegende Erfindung betrifft die Verwendung von an sich bekannten wasserlöslichen Polyetherpolyolen, sowie deren an den Endgruppen mit Alkyl- bzw. Acylgruppen substituierten Derivaten als antitumoral wirkende Mittel.

Es ist schon bekannt geworden, daß copolymere, komplexe $Cu^{II}$ - und $Co^{II}$-Salze der Ethylenmaleinsäure gegen das Walker-Sarkom wirksam sind /J. Med. Chem. 12 (1969), 1180/.

Weiterhin wurden Polykationen verschiedenen Typs, z.B. Polyamidamine, Poly-N-morpholinoethylacrylamid und N-Oxid-Polymere auf Hemmung der Metastasierung geprüft mit dem Ergebnis, daß nur die Tumorzellaussaat, nicht aber das Metastasenwachstum in situ bzw. Lymphknotenmetastasen beeinflußt werden konnten /J. Med. Chem. 16 (1973), 496/.

Ferner wurde die Wirksamkeit von Polymeren mit Carboxylgruppen gegen das Sarkom 180 in Abhängigkeit vom Molekulargewicht, der Ladungsdichte sowie der Metall-Bindungsfähigkeit der Carboxylgruppen beschrieben /Dissertation Abstr. Intern. B 33 (1973), 5745/.

Polyanionen, z.B. Poly-ammoniumacrylat, Acrylsäure-acrylamid-copolymerisate sowie Copolymere aus Ethylen- und Maleinsäureanhydrid sollen im Zusammenhang mit ihrer Antitumorwirkung einen Heparin-ähnlichen Effekt, daneben eine Virushemmung besitzen und außerdem die Immunreaktionen steigern /J. Med. Chem. 17 (1974), 1335/.

Aus allen diesen Arbeiten geht hervor, daß die Antitumorwirkung der bisher untersuchten Polymeren an den verwendeten experimentellen Tumoren oft nur an der unteren Grenze der Signifikanz liegt und verschiedentlich nur auf prophylaktische oder adjuvantive Effekte beschränkt ist. Nachteilig erscheint außerdem, daß die zitierten Untersuchungen vielfach an allogenen, zur Spontanregression neigenden Mäusetumoren und nicht systematisch und unter kliniknahen Versuchsanordnungen durchgeführt wurden. Meist fehlen Angaben zur Toxizität der Präparate, obwohl die Applikation hoher Dosierungen von Substanzen mit einem Molekulargewicht von über 30 000 eine mangelhafte Ausscheidung bzw. eine Speicherung in den Geweben vermuten läßt.

W. Regelson et. al. /Nature 186 (1960) 778-780/ untersuchten die Antitumorwirkung synthetischer Polyelektrolyte, wie Polyacrylsäure, Polymethacrylsäure und hydrolysierten bzw. aminolysierten Ethylen-Maleinsäureanhydrid-Copolymerisaten. Sie fanden durch Wirkungsvergleich der Dicarbonsäure-, der Amid-Carbonsäure- und der Diamid-Form von Ethylen-Maleinsäureanhydrid-Copolymerisaten, daß für eine signifikante Tumorinhibierung mindestens eine ionisierbare Carboxylgruppe erforderlich ist. Versuche dieser Autoren mit Polyacrylamiden ergaben in hohen Dosen (800 mg/kg, MW 60 - 70 000 und 400 mg/kg, MW 120 000) eine negative, bzw. eine nicht signifikante sehr geringe positive Tumorhemmwirkung.

Ebenfalls wurden antitumoral wirkende Mittel beschrieben, die durch einen Gehalt an mindestens einem wasserlöslichen Homo- oder Copolymerisat, das das 1,3-Dihydroxy-2-methylenpropan und/oder dessen Derivate enthält, charakterisiert sind /DOS 2 705 189/. Ähnliche Präparate mit ähnlicher Wirkung sind wasserlösliche Homo- oder Copolymerisate, die 3,4-Dihydroxibuten-1 oder Hydroxi-alkyl-(meth)-acrylate sowie deren Derivate oder auch Derivate des Allylalkohols polymerisiert oder copolymerisiert enthalten /DOS 27 40 082/.

Außerdem ist eine gewisse antitumorale Wirkung von Emulgatoren, die Polyethylenoxidketten eingebaut enthalten, bekannt geworden. So wurde Polyoxiethylen-Sorbitanmonooleat (Tween 80) für die Immunisierung gegen den hyperdiploiden Ehrlich-Tumor verwendet /Experientia 29 (1973) 710/. Ein Blockcopolymerisat aus Poly-oxypropylen und Polyoxyethylen (Pluronic F 68) erwies sich als wirksam gegen den Metastasenangang des Walker 256 Ascites Tumors, wahrscheinlich durch Beeinflussung der Blutgerinnungsfähigkeit /Cancer 29 (1972), 171/. Diese Präparate sind bekanntermaßen hochwirksame Emulgatoren und sind aus diesem Grunde nicht sehr gut verträglich, insbesondere bei parenteraler Applikation.

Es wurde nun überraschenderweise gefunden, daß Polyetherpolyole sowie teilweise oder vollständig an den endständigen Hydroxylgruppen durch Alkyl- oder Acylgruppen mit jeweils maximal 8 C-Atomen substituierte Polyetherpolyole starke antitumorale Eigenschaften besitzen.

Die Substanzen zeigen im Molekulargewichtsbereich von etwa 400 bis über 20 000 signifikante prophylaktische und kurative Wirkungen an soliden Tumoren syngener Systeme in einem breiten Dosisbereich von 0,5 bis 500 mg/kg, vorzugsweise 5 bis 250 mg/kg, unter kliniknahen Versuchsanordnungen und Applikationsarten. Die akute Toxizität gereinigter Substanzen ist gering, die LD 50 liegt bei der intravenösen (i.v.) Applikation meist oberhalb 2500 mg/ kg, so daß die Substanzen eine ungewöhnlich große therapeutische Breite besitzen. Die erfindungsgemäßen Mittel stellen somit eine wichtige Bereicherung der Therapie dar.

Die erfindungsgemäß zu verwendenden Polyetherpolyole und Derivate von Polyetherpolylen besitzen die allgemeine Formel

$$R\left[\left(O-\overset{\overset{A}{|}}{C}H-CH_2\right)_n-OR'\right]_m$$

in welcher

A = statistisch verteilt H oder $CH_3$, wobei maximal 50 % der A-Gruppen $CH_3$ sind,

n = Zahlen von 2 bis 500,
m = Zahlen von 2 bis 8,
R = Rest eines Diols oder Polyols mit bis zu 8 OH-Gruppen und 2-12 C-Atomen,

die Reste R' gleich oder verschieden sind, und

Wasserstoff, einen aliphatischen, alicyclischen, aromatischen oder heterocyclischen Rest mit 1-8 C-Atomen, einen Rest
X-CO- mit X = Wasserstoff, aliphatischer, alicyclischer oder aromatischer Rest mit 1-7 C-Atomen, einen Rest
Y-O-CO- mit Y = aliphatischer, cycloaliphatischer, aromatischer oder heterocyclischer Rest mit 1-7 C-Atomen, oder einen Rest
Z-NH-CO- mit Z = Wasserstoff, aliphatischer, cycloaliphatischer, aromatischer oder heterocyclischer Rest mit 1-7 C-Atomen
bedeuten.

Bevorzugt als Wirkstoffe für die erfindungsgemäßen Mittel sind Polyetherdiole oder -triole der allgemeinen Formel II

$$R\left[-\left(O-\overset{A}{CH}-CH_2\right)_n-OH\right]_m \qquad II$$

mit
R = Rest eines Diols oder Triols mit 2-8 C-Atomen, besonders bevorzugt $-CH_2-CH_2-$ und $-CH_2-\underset{CH_3}{CH}-$ ;

A = statistisch verteilt H oder $CH_3$, wobei maximal 50 %, vorzugsweise weniger als 33 % der A-Gruppen $CH_3$ sind;

n = 2-500, vorzugsweise 3-250 und

m = 2 oder 3;

endständig alkylierte Polyetherdiole und -triole der Formel III

$$R\left[\left(O-\overset{A}{\overset{|}{C}}H-CH_2\right)_n-OR'\right]_m \qquad III,$$

in der R, A, n und m die gleiche Bedeutung besitzen wie in Formel II und die Reste R' gleich oder verschieden H, $C_1$-$C_8$-Alkyl-, Cycloalkyl- oder Aryl-, vorzugsweise H oder $C_1$-$C_4$-Alkyl sein können;

endständig acylierte Polyetherdiole oder -triole der Formel IV

$$R\left[\left(O-\overset{A}{\overset{|}{C}}H-CH_2\right)_n-O-\overset{O}{\overset{\|}{C}}-R'\right]_m \qquad IV,$$

in der R, A, n und m die gleiche Bedeutung besitzen wie in Formel II und die Reste R' gleich oder verschieden H oder Alkyl-, Cycloalkyl- oder Arylgruppen mit 1-7 C-Atomen, vorzugsweise H oder $C_1$-$C_3$-Alkylgruppen sein können;

Verbindungen der Formel V,

$$R\left[ -\!(O-\overset{A}{\overset{|}{C}}H-CH_2)_n\!-O-\overset{O}{\overset{\|}{C}}-O-R' \right]_m \qquad V,$$

in der R, A, n und m die gleiche Bedeutung besitzen wie in Formel II und die Reste R' gleich oder verschieden eine Alkyl-, Cycloalkyl- oder Arylgruppe mit 1-7 C-Atomen, vorzugsweise $C_1$-$C_4$-Alkylgruppen sein können;

sowie Verbindungen der Formel VI

$$R\left[ -\!(O-\overset{A}{\overset{|}{C}}H-CH_2)_n\!-O-\overset{O}{\overset{\|}{C}}-NHR' \right]_m \qquad VI,$$

in der R, A, n und m die gleiche Bedeutung besitzen, wie in Formel IIund die Reste R' gleich oder verschieden H oder Alkyl-, Cycloalkyl- oder Arylgruppen mit 1-7 C-Atomen, vorzugsweise H oder $C_1$-$C_4$-Alkylgruppen sein können.

Polyetherpolyole sind dem Fachmann bekannt. Sie werden in großen Mengen zur Herstellung von Polyurethankunststoffen verwendet. Ihre Herstellung erfolgt durch ringöffnende Polymerisation oder Copolymerisation von Ethylenoxid und Propylenoxid. Wird die Polymerisation der Alkylenoxide mit Alkali- oder Erdalkalimetallhydroxiden gestartet, so erhält man Polyalkylenglykole mit zwei OH-Endgruppen. (Houben-Weyl, Methoden der org. Chemie, 4. Auflage, Bd. XIV/2, 1963, S. 427ff., Ullmanns Encylopädie der technischen Chemie, 3. Auflage, Bd. 14 (1963), S. 49-52). Ausgehend von Ethylenoxid erhält man die Polyethylenglykole, die vom Diethylenglykol bis zu Produkten mit Molekulargewichten über $10^6$ sehr gut in Wasser löslich sind. Von den aus Propylenoxid entstehenden Polypropylenglykolen sind nur die relativ niedermolekularen Vertreter in Wasser löslich. Statistische Copolymere von Ethylenoxid und Propylenoxid sind je nach ihrem Molekulargewicht und Gehalt an Propylenoxidbausteinen mehr oder weniger gut in Wasser löslich.

Startet man die Polymerisation der Alkylenoxide mit Alkalimetallalkoholaten von Triolen oder Polyolen, so erhält man Polyethertriole bzw. -polyole, die soviele OH-Gruppen besitzen wie das Ausgangspolyol.

Ausgehend von Alkoholaten einwertiger Alkohole erhält man Polyalkylenoxide mit einer Ether- und einer freien OH-Gruppe.

Die Hydroxylendgruppen der Polyetherpolyole sowie der Polyalkylenglykolmonoalkylether können nach bekannten Methoden in Ethergruppen, Carbonsäureestergruppen, Kohlensäureestergruppen oder Urethangruppen überführt werden.

Als Alkylierungsmittel zur Veretherung der Hydroxylgruppen seien beispielsweise Dialkylsulfate, Alkylhalogenide sowie Ester der p-Toluolsulfonsäure genannt,

/Organikum, Organisch-chemisches Grundpraktikum, 6. Auflage, Berlin 1967, S. 185 ff_7. Die Einführung von Carbonsäureestergruppen kann beispielsweise durch Einwirkung von Säureanhydriden in Gegenwart eines sauren Katalysators, oder durch Umsetzung mit Säurechloriden in Gegenwart eines tertiären Amins erfolgen. Kohlensäureestergruppen können durch Umsetzung mit Chlorameisensäureestern in Gegenwart inerter Basen hergestellt werden.

Die Umsetzung mit Monoisocyanaten in wasserfreiem Medium sowie gegebenenfalls in Gegenwart von Katalysatoren, wie Zinnverbindungen oder tertiären Aminen führt zur Einführung von Urethanendgruppen. Die Einführung unsubstituierter Carbamatgruppen kann durch Umsetzung der mit überschüssigem Phosgen erhältlichen Chlorameisensäureester mit Ammoniak erfolgen.

Zweckmäßigerweise werden die erfindungsgemäß zu verwendenden Polyetherpolyole bzw. Polyetherpolyolderivate vor der Herstellung der erfindungsgemäßen antitumoralen Mittel sorgfältig gereinigt. Flüssige Produkte können beispielsweise mehrfach oder kontinuierlich mit frisch destilliertem Diethylether extrahiert werden. Kristalline Produkte, wie beispielsweise Polyethylenglykole oder Derivate von Polyethylenglykolen können durch gegebenenfalls mehrfaches Umkristallisieren, z.B. aus einem Gemisch von Ethylacetat und Diethylether gereinigt werden.

Die erfindungsgemäß zu verwendenden Polyetherpolyole bzw. Derivate von Polyetherpolyolen sind in physiologischer

Kochsalzlösung bei Temperaturen von 20-40°C zu mindestens 0,5 Gew.-% löslich. Ihr Molekulargewicht beträgt besonders im Hinblick auf eine mögliche Ausscheidung nach parenteraler Applikation vorzugsweise etwa 300-30 000. Besonders bevorzugt sind wegen ihrer definierten und einheitlichen Struktur die Polyethylenglykole und ihre an den endständigen Hydroxylgruppen substituierten Derivate.

Die erfindungsgemäßen Mittel haben bei äußerst niedriger Toxizität eine starke antitumorale Wirkung gegen tierische und menschliche Tumoren und sollen daher zur Bekämpfung von durch Tumoren verursachten Erkrankungen verwendet werden.

Die erfindungsgemäßen Mittel werden hergestellt, indem man die Wirkstoffe in physiologischer Kochsalzlösung löst. Sie können aber auch in Tabletten, Kapseln, Säfte und andere Zubereitungen für die orale Applikation eingearbeitet werden. Die dabei erhaltenen Zubereitungen werden intraperitoneal, intravenös, intramuskulär oder oral appliziert. Der therapeutische Dosisbereich reicht von 0,5 bis 500 mg/kg, vorzugsweise 5 bis 250 mg/kg. Die ungewöhnliche Breite dieses Bereiches beruht auf der ungewöhnlich geringen Toxizität der Wirkstoffe.

Die Substanzen wurden in zahlreichen Versuchen unter verschiedenen Testbedingungen am Carcinom EO 771 bzw. am Mäusesarkom MCS 4 auf die Induktion von Tumorwirkungen geprüft.

Die Methodik der Untersuchungen an diesen beiden experimentellen Tumoren ist in den Versuchsbeschreibungen (a) und (b) enthalten.

Versuchsbeschreibung a)

TUMOR-TESTE AM CARCINOM EO 771 AUF C. 57 BL/6 - MÄUSEN

Tierart: C. 57 BL/6 - Mäuse, Inzucht (SPF)

Verfahren: Stammhaltung: Am 14.-20. Tag nach letzter Transplantation subcutane Verimpfung einer Zellsuspension des Carcinoms EO 771 in 0,5 ml Phosphat-gepufferter 0,9 %iger NaCl-Lösung auf C. 57 BL/6 - Mäuse

Vorbereitung von Screening-Testen: Gleiches Verfahren wie bei Stammhaltung des Tumors, jedoch subcutane Verimpfung einer Suspension von 5 x $10^4$ Tumorzellen in 0,5 ml einer Phosphat-gepufferten 0,9 %igen NaCl-Lösung.

Behandlung: Einmalige, intramuskuläre Injektion der geforderten Präparatlösungen am 6. Tag vor bzw. am 2. Tag nach der Tumortransplantation.

Versuchsdauer: 18 - 22 Tage nach der Tumortransplantation. Dann Abtötung der Tiere, Präparation und Wägung der subcutanen Tumoren.

0044959

Auswertungsparameter: Hemmung des Tumorwachstums durch Bestimmung des durchschnittlichen Tumorgewichts von Kontrollen und behandelten Tiergruppen sowie Errechnung des Tumorgewichts- (TG-)-Index nach der Formel:

$$\text{TG-Index} = \frac{\text{Ø Tumorgewicht der behandelten Tiergruppen}}{\text{Ø Tumorgewicht der Kontrollgruppe}}$$

Beurteilung der Testergebnisse:
TG-Index 0,8 - 0,6 = Wirkungsandeutung;
0,6 - 0,4 = deutliche Wirkung;
0,4 = gute Wirkung.

Versuchsbeschreibung b)

TUMOR-TESTE AM SARKOM MCS 4 AUF C 57 BL/6 - MÄUSEN

Tierart: C 57 BL/6 - Mäuse, Inzucht (SPF)

Verfahren: Stammhaltung: 10 - 14 Tage nach letzter Transplantation subcutane Verimpfung einer Tumorsuspension des Sarkoms MCS 4 in 0,5 ml einer Phosphat-gepufferten 0,9 %igen NaCl-Lösung auf C 57 BL/6 - Mäuse.

Vorbereitung von Screening-Testen:
Gleiches Verfahren wie bei Stammhaltung des Tumors, jedoch subcutane Verimpfung einer Suspension von 2 x $10^5$ Tumorzellen in 0,5 ml

einer Phosphat-gepufferten 0,9 %igen NaCl-Lösung.

<u>Behandlung:</u> Einmalige, intravenöse Injektion der geforderten Präparatlösungen am 2. Tag vor, bzw. am 2. Tag nach der Tumortransplantation.

<u>Versuchsdauer:</u> 18 - 22 Tage nach der Tumortransplantation. Dann Abtötung der Tiere, Präparation und Wägung der Tumoren.

<u>Auswertung und Beurteilung der Ergebnisse</u> erfolgen analog wie in der Versuchsbeschreibung a).

Tabelle 1

Prüfergebnisse von Polyethylenglykolen am Carcinom EO 771 bei einmaliger i.m-Applikation.

| Molekulargewicht $\bar{M}_n$ des Polyethylenglykols | Dosis [mg]/kg | Behandlungstag+ | Tumorgewichts-Index |
|---|---|---|---|
| 400 | 250 | - 6 | 0,41 |
| | 250 | + 2 | 0,33 |
| 2.000 | 250 | - 6 | 0,38 |
| | 250 | + 2 | 0,44 |
| 4.000 | 2,5 | - 6 | 0,21 |
| | 10 | + 2 | 0,25 |
| 12.000 | 50 | - 6 | 0,19 |
| | 10 | + 2 | 0,32 |
| 20.000 | 100 | - 6 | 0,35 |
| | 100 | + 2 | 0,41 |

+ Behandlungstag: - 6 = 6 Tage vor Tumortransplantation
+ 2 = 2 Tage nach Tumortransplantation

Tabelle 2

Prüfergebnisse von Derivaten von Polyethylenglykolen am Carcinom EO 771 bzw. am Sarkom MCS 4.

| Präparat aus Beispiel | Test-system | Dosis /mg/kg/ | Applikation | Behand-lungs-tag* | Tumor-gewichts-Index |
|---|---|---|---|---|---|
| 1 | EO 771 | 250 | 1 x i.m. | - 6 | 0,43 |
| | | 50 | 1 x i.m. | + 2 | 0,51 |
| 2 | EO 771 | 2,5 | 1 x i.m. | - 6 | 0,42 |
| | | 0,5 | 1 x i.m. | + 2 | 0,31 |
| 3 | EO 771 | 50 | 1 x i.m. | - 6 | 0,31 |
| | | 250 | 1 x i.m. | + 2 | 0,48 |
| 4 | MCS 4 | 10 | 1 x i.v. | - 2 | 0,41 |
| | | 50 | 1 x i.v. | + 2 | 0,28 |
| 5 | EO 771 | 100 | 1 x i.m. | - 6 | 0,34 |
| | | 50 | 1 x i.m. | + 2 | 0,40 |

* Behandlungstag: - 6 = 6 Tage vor Tumortransplantation
+ 2 = 2 Tage nach Tumortransplantation

Die Tumorgewichts-Indices der aufgeführten Präparate in den Tabellen 1 und 2 lassen erkennen, daß die Substanzen sowohl am Carcinom EO 771 als auch am Sarkom MCS 4 bei verschiedenen Dosierungen und Applikationswegen sowie an verschiedenen Behandlungstagen deutliche Tumorhemmwirkungen zu induzieren vermögen.

Beispiele

Die geprüften Polyethylenglykole waren handelsübliche technische Produkte, die im Falle des Polyethylenglykols mit dem mittleren Molekulargewicht $\bar{M}_n$ = 400 durch fünfmaliges Ausschütteln mit dem gleichen Volumen frisch destillierten Diethylethers, im Falle der kristallinen Polyethylenglykole mit $\bar{M}_n$ = 2.000, 4.000, 12.000 und 20.000 durch zweimaliges Umkristallisieren aus Ethylacetat/Diethylether 2:1 gereinigt wurden.

Beispiel 1

$$CH_3CO-O-[CH_2CH_2-O]_{45}-CO-CH_3$$

200 g eines Polyethylenglykols mit einem mittlerem Molekulargewicht von 2.000 werden mit 400 ml destilliertem Toluol versetzt. Zur Trocknung werden bei Normaldruck 150 ml Toluol abdestilliert. Sobald die Lösung auf 80°C abgekühlt ist, werden 0,5 g p-Toluolsulfonsäure zugegeben, und innerhalb 30 Minuten werden 40 g Essigsäureanhydrid zugetropft. Anschließend wird 2 Stunden zum Sieden erhitzt, das Lösungsmittel im Vakuum am Rotationsverdampfer entfernt und der Rückstand zweimal aus Ethylacetat/Diethylether 1:1 umkristallisiert und im Ölpumpenvakuum bei Raumtemperatur getrocknet.

Ausbeute: 162 g Polyethylenglykoldiacetat

OH-Zahl = 0,7; Säurezahl = 1,1

Beispiel 2

$$CH_3-CH_2-CH_2-CH_2-O-[CH_2-CH_2-O]_{20,6}-H$$

148 Gew.-Teile n-Butanol wurden mit 2 Gew.-Teilen pulverförmigem NaOH verrührt und auf 80 - 100°C erwärmt. Bei dieser Temperatur wurde Ethylenoxid eingeleitet, bis eine Gewichtszunahme von 2.444 Gewichtsteilen festgestellt wurde. Es wurde 1 Stunde bei 100°C im Vakuum nachgerührt. Das Reaktionsprodukt wurde in 10. 000 Vol.-Teilen Toluol gelöst und mit gasförmiger HCl neutralisiert. Das ausgefallene NaCl wurde abgesaugt und das Toluol im Vakuum wieder abgedampft. Die Verbindung besaß ein Molekulargewicht $\bar{M}_n$ = 982 (bestimmt durch Dampfdrucksomometrie in DMF).

Beispiel 3

Diurethan der Formel

$$CH_3-NH-CO-O-[CH_2-CH_2-O]_{45}-CONHCH_3$$

100 g Polyethylenglykol mit einem mittleren Molekulargewicht $\bar{M}_n$ von 2.000 werden mit 200 ml Toluol versetzt. Zum Trocknen werden 75 ml Toluol unter Normaldruck abdestilliert. Nach dem Abkühlen werden bei Raumtemperatur 9 g Methylisocyanat und 50 mg Zinnoctanoat zugegeben und die Mischung im Verlauf von 2 Stunden bis zum Sieden erhitzt und 2 Stunden unter Normaldruck abdestilliert. Nach dem Abkühlen wird das Reaktionsprodukt durch Zugabe von 200 ml Diethylether ausgefällt, abgesaugt, mit Diethylether gewaschen und aus Ethylacetat/Diethylether 1:1 umkristallisiert.

Ausbeute: 99 g einer kristallinen, gut wasserlöslichen Substanz. OH-Zahl = 1,0.

Beispiel 4

Diurethan der Formel

$$n\text{-}C_4H_9\text{-}NH\text{-}CO\text{-}O\text{-}[CH_2\text{-}CH_2\text{-}O]_{135}\text{-}CO\text{-}NH\text{-}C_4H_9$$

600 g eines Polyethylenglykols mit mittlerem Molekulargewicht 6.000 werden in möglichst wenig Chloroform bei Siedetemperatur gelöst. 20 g n-Butylisocyanat werden innerhalb 30 Minuten zugetropft und 2,5 Stunden unter Rückfluß nachgerührt. Das Chloroform wird abgedampft und der Rückstand zweimal aus Ethylacetat/Diethylether 1:1 umkristallisiert. Man erhält 537 g einer kristallinen Substanz, die sich leicht in Wasser löst.

OH-Zahl = 1,2

Beispiel 5

$$C_2H_5\text{-}O\text{-}CO\text{-}O\text{-}[CH_2\text{-}CH_2\text{-}O]_{135}\text{-}CO\text{-}O\text{-}C_2H_5$$

100 g eines Polyethylenglykols mit mittlerem Molekulargewicht 6.000 werden in 1 l Toluol gelöst. Zur Entfernung von Wasserspuren werden 200 ml Toluol unter Normaldruck abdestilliert. Nach dem Abkühlen auf Raumtemperatur werden 6 g trockenes Pyridin zugegeben und anschließend 7 g Chlorameisensäureethylester in 200 ml Toluol zugetropft. Es wird 5 Stunden bei Raumtemperatur nachgerührt, das ausgefallene Pyridinhydrochlorid abfiltriert, das Toluol im Vakuum abdestilliert und der Rückstand zweimal aus Ethylacetat/Diethylether 2:1 umkristallisiert.

Ausbeute: 83 g, OH-Zahl = 1,0

Patentansprüche:

1. Antitumoral wirkende Mittel, gekennzeichnet durch einen Gehalt an mindestens einem wasserlöslichen Polyetherpolyol oder einem teilweise oder vollständig an den endständigen Hydroxylgruppen durch Alkylgruppen oder Acylgruppen mit jeweils maximal 8 C-Atomen substituierten Polyetherpolyol der allgemeinen Formel

$$R\left[\left(O-\overset{A}{\overset{|}{C}}H-CH_2\right)_n-OR'\right]_m$$

in welcher

A - statistisch verteilt H oder $CH_3$, wobei maximal 50 % der A-Gruppen $CH_3$ sind,

n - Zahlen von 2 bis 500,

m - Zahlen von 2 bis 8,

R - Rest eines Diols oder Polyols mit bis zu 8 OH-Gruppen und 2-12 C-Atomen,

die Reste R', gleich oder verschieden, Wasserstoff, einen aliphatischen, alicyclischen, aromatischen oder heterocyclischen Rest mit 1-8 C-Atomen, einen Rest

X-CO- mit X = Wasserstoff, aliphatischer, alicyclischer oder aromatischer Rest mit 1-7 C-Atomen, oder einen Rest

Y-O-CO- mit Y = aliphatischer, cycloaliphatischer, aromatischer oder heterocyclischer Rest mit 1-7 C-Atomen, oder einen Rest

Z-NH-CO mit Z = Wasserstoff, aliphatischer, cycloaliphatischer, aromatischer oder heterocyclischer Rest mit 1-7 C-Atomen,

bedeuten.

2. Antitumoral wirkende Mittel, gekennzeichnet durch einen Gehalt an mindestens einem Polyetherdiol oder Polyethertriol der allgemeinen Formel II

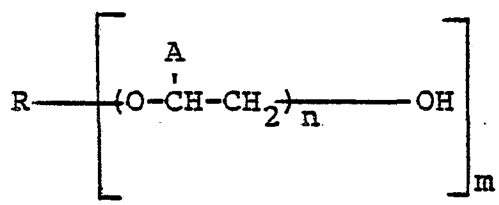

II

mit

R = Rest eines Diols oder Triols mit 2-8 C-Atomen,

A = statistisch verteilt H oder $CH_3$, wobei maximal 50 % der A-Gruppen $CH_3$ sind,

n = Zahlen von 2 bis 500 und

m = 2 oder 3.

3. Antitumoral wirkende Mittel, gekennzeichnet durch einen Gehalt an mindestens einem endständig alkylierten Polyetherdiol oder Polyethertriol der allgemeinen Formel III

$$R-\left[-(O-\overset{A}{\overset{|}{C}}H-CH_2)_n-OR'\right]_m \qquad III$$

in der R, A, n und m die gleiche Bedeutung besitzen wie in Formel II und die Reste R' gleich oder verschieden H oder $C_1$-$C_4$-Alkyl sein können.

4. Antitumoral wirkende Mittel, gekennzeichnet durch einen Gehalt an mindestens einem endständig acylierten Polyetherdiol oder Polyethertriol der allgemeinen Formel IV

$$R-\left[-(O-\overset{A}{\overset{|}{C}}H-CH_2)_n-O-\overset{O}{\overset{\|}{C}}-R'\right]_m \qquad IV$$

in der R, A, n und m die gleiche Bedeutung besitzen wie in Formel II und die Reste R' gleich oder verschieden H oder $C_1$-$C_3$-Alkylgruppen sein können;

5. Antitumoral wirkende Mittel, gekennzeichnet durch einen Gehalt an mindestens einem endständig acylierten Polyetherdiol oder Polyethertriol der allgemeinen Formel V

$$R-\left[-(O-\overset{A}{\overset{|}{C}}H-CH_2)_n-O-\overset{O}{\overset{\|}{C}}-O-R'\right]_m \qquad V$$

in der R, A, n und m die gleiche Bedeutung besitzen wie in Formel II und die Reste R' gleiche oder verschiedene $C_1$-$C_4$-Alkylgruppen sein können.

6. Antitumoral wirkende Mittel, gekennzeichnet durch einen Gehalt an mindestens einem endständig acylierten Polyetherdiol oder Polyethertriol der allgemeinen Formel VI

$$R-\left[(O-\overset{A}{\overset{|}{C}H}-CH_2)_n-O-\overset{O}{\overset{\|}{C}}-NHR'\right]_m \qquad VI$$

in der R, A, n und m die gleiche Bedeutung besitzen, wie in Formel II und die Reste R' gleich oder verschieden H oder $C_1$-$C_4$-Alkylgruppen sein können.

7. Antitumoral wirkende Mittel nach den Ansprüchen 1-6, dadurch gekennzeichnet, daß A in den allgemeinen Formeln Wasserstoff bedeutet.

8. Antitumoral wirkende Mittel nach den Ansprüchen 1-7, dadurch gekennzeichnet, daß in den allgemeinen Formeln R = $-CH_2-CH_2-$ und m = 2 ist.

9. Verfahren zur Herstellung eines antitumoral wirkenden Mittels, dadurch gekennzeichnet, daß man mindestens ein wasserlösliches Polyetherpolyol oder endständig substituiertes Polyetherpolyol gemäß den Ansprüchen 1-8 in einer physiologischen Kochsalzlösung löst, oder in Zubereitungen für die orale Applikation einarbeitet.

10. Verfahren zur Behandlung von Tumoren, dadurch gekennzeichnet, daß man dem Organismus von an Tumoren erkrankten Menschen antitumoral wirkende Mittel gemäß den Ansprüchen 1 - 8 systemisch oder lokal appliziert.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0044959

Nummer der Anmeldung

EP 81 10 5048

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CHEMICAL ABSTRACTS, Band 70, Nr. 23, 9. Juni 1969, Zusammenfassung Nr. 104991k, Seite 198 Columbus, Ohio, U.S.A. F. HARTVEIT: "Inhibition of the subcutaneous growth of Ehrlich's ascites carcinoma in female mice by polyethylene glycol 4000" & J. PATHOL. 1969, 97(1), 145-148 * Zusammenfassung * | 1,2,7-9 |
| | -- | |
| X | L.G. DONARUMA: "Polymeric drugs", 1978, Seiten 240-245 Academic Press New York, U.S.A. * Seiten 240,245 * | 1,2,7,8 |
| | -- | |
| A | DE - A - 2 904 407 (C. ROTH) * Ansprüche 1,2 * | 1,2,7,8 |
| | -- | |
| A | FR - A - 6 973 (UNIMED) * Zusammenfassung * | 1 |

**KLASSIFIKATION DER ANMELDUNG (Int. Cl.³)**

A 61 K 31/77
31/785

**RECHERCHIERTE SACHGEBIETE (Int. Cl.³)**

A 61 K 31/77
31/785
31/74

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9

Unvollständig recherchierte Patentansprüche:

Nicht recherchierte Patentansprüche: 10: Verfahren zur chirurgischen oder therapeutischen Behandlung des menschlichen oder tierischen Körpers. (Siehe Art. 52(4) des Europäischen Patentübereinkommens).

Grund für die Beschränkung der Recherche:

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 09-11-1981 | PELTRE |